# EUROPEAN PATENT SPECIFICATION

(11) **EP 3 853 322 B1**
(45) Date of publication and mention of the grant of the patent: **19.04.2023**
(21) Application number: 19779397.9
(22) Date of filing: 19.09.2019
(51) Int. Cl.: C09K 11/02, C09K 11/06, G01N 21/64

(54) **LIGHT-EMITTING PARTICLE**
LICHTEMITTIERENDES TEILCHEN
PARTICULE ÉLECTROLUMINESCENTE

(30) Priority: 20.09.2018 GB 201815329
(43) Date of publication of application: 28.07.2021
(73) Proprietor: Sumitomo Chemical Company Limited, Tokyo 104-8260 (JP)
(72) Inventor: BEHRENDT, Jonathan, Cambridgeshire PE29 2XG (GB); KAMTEKAR, Kiran, Cambridgehsire Cambridgeshire PE29 2XG (GB)
(74) Representative: Gilani, Anwar
(86) International application number: PCT/EP2019/075235
(87) International publication number: WO 2020/058440

(56) References cited:
- WO-A2-2011/057295
- GB-A- 2 554 666
- US-A1- 2004 018 379
- US-A1- 2008 293 584
- US-A1- 2012 267 585
- REN ZHONGJIE ET AL: "Polysiloxanes for optoelectronic applications", PROGRESS IN MATERIALS SCIENCE, PERGAMON PRESS, GB, vol. 83, 19 July 2016 (2016-07-19), pages 383-416, XP029773970, ISSN: 0079-6425, DOI: 10.1016/J.PMATSCI.2016.07.004

## Description

### Background

Embodiments of the present disclosure relate to light-emitting particles, in particular light-emitting particles, and the use thereof as a luminescent marker. Embodiments of the present disclosure further relate to methods of preparing said light-emitting particles.

### Background

Nanoparticles of silica and a light-emitting material have been disclosed as labelling or detection reagents.

Nanoscale Res. Lett., 2011, vol. 6, p 328 discloses entrapment of a small molecule in a silica matrix.

Langmuir, 1992, vol. 8, pp 2921-2931 discloses coupling of a dye to a silane coupling agent which is then incorporated into a silica sphere.

J. Mater. Chem., 2013, vol. 1, pp 3297-3304, Behrendt et al. describes silica-LEP nanoparticles where the LEP is covalently bound to the silica. The light emitting polymer has alkoxysilane groups pendant from the polymer backbone which react with the silica monomer during formation of the nanoparticles.

Nanoscale, 2013, vol. 5, pp 8593-8601, Geng et al. describes silica-conjugated polymer (CP) nanoparticles wherein the LEP has pendant non-polar alkyl side chains and where the nanoparticles have a "SiO₂@CP@SiO₂" structure.

Chem. Mater., 2014, vol. 26, pp 1874-1880, Geng et al. discloses poly(9,9-dihexylfluorene-*alt*-2,1,3-benzothiadiazole) (PFBT) loaded nanoparticles.

GB2554666 discloses a composite particle comprising a mixture of a silica polymer and a light-emitting polymer comprising a backbone and polar groups pendant from the backbone.

US2012267585 discloses compositions comprising nanoparticles in which at least one observable marker, such as a radioisotope or fluorophore, is incorporated within the nanosized object.

US2008293584 discloses colloidal silica particles containing a fluorescent dye compound, composed of a silica particle containing a silica component and a fluorescent dye compound chemically bound or adsorbed thereto, wherein the colloidal silica particles containing a fluorescent dye compound have an average diameter of 30 nm or less, and wherein said silica particles are used simultaneously as fluorescent-labelling nanobeads.

WO2011057295 discloses functionalized chromophoric polymer dots comprising a hydrophobic core and a hydrophilic cap, and bioconjugates thereof.

### Summary

The present inventors have found that a light-emitting polymer may be provided in a high concentration in a light-emitting particle with little or no adverse effect on brightness. This may allow formation of an ultrabright particle containing the light-emitting polymer and another light-emitting material.

There is provided a particle according to claim 1.

The present inventors have found that a non-polymeric light-emitting material in a particle may be susceptible to being washed out of the particle when dispersed in a liquid, *e.g.* a buffer solution. The present inventors have found that providing an ionic non-polymeric light-emitting material and an ionic light-emitting polymer with opposing ionic charges in a particle may allow for formation of a stable colloid of the particles whilst preventing the non-polymeric light-emitting material from being washed out of the particle.

The opposing ionic charges of the non-polymeric light-emitting material and the light-emitting polymer and / or confinement of these light-emitting materials within a particle may cause these light-emitting materials to be brought into close proximity during particle formation, resulting in particles in which the proximity of these light-emitting materials allows for efficient energy transfer, such as Förster resonance energy transfer (FRET), from the light-emitting polymer to the non-polymeric light-emitting material.

Accordingly, the first light-emitting material has one of a net positive and net negative ionic charge, and the second light-emitting material has the other of net positive or net negative ionic charge.

The particles may be dispersed in a liquid, e.g. for use in an assay.

Accordingly, in some embodiments there is provided a colloidal suspension in which the particles are suspended in a liquid.

The inorganic matrix may be polymeric. In some embodiments, the particle may be formed by polymerising a monomer for forming a polymeric matrix, e.g. a silica monomer, in the presence of the first light-emitting material and the second light-emitting material.

The particles may be used for marking a biomolecule, e.g. to track or detect the biomolecule. Accordingly, in some embodiments there is provided a method of marking a biomolecule comprising the step of binding the biomolecule to the particle.

In some embodiments, there is provided an assay method for a target analyte in which a sample is contacted with light-emitting marker particles as described herein, and determining any binding of the target analyte to the light-emitting marker.

Optionally, the sample contacted with the light-emitting marker particles is analysed by flow cytometry.

Optionally, an amount of target analyte bound to the light-emitting marker particles is determined.

Optionally, the sample comprises a mixture of cells and one or more different types of target cells bound to the light-emitting marker are identified and / or quantified.

### Description of the Drawings

The disclosed technology and accompanying figures describe some implementations of the disclosed technology.
Figure 1 is a schematic illustration of a light-emitting particle having a polymeric light-emitting compound which, upon excitation, transfers energy to a non-polymeric light-emitting compound;
Figure 2 is graphs of absorption spectra (dotted line) and emission spectra (solid line) spectra at different non-polymeric light-emitter: light-emitting polymer mass ratios; and
Figure 3 is graphs of nanoparticle diameters for different non-polymeric light-emitter: light-emitting polymer mass ratios.

The drawings are not drawn to scale and have various viewpoints and perspectives. The drawings are some implementations and examples. Additionally, some components and/or operations may be separated into different blocks or combined into a single block for the purposes of discussion of some of the embodiments of the disclosed technology. Moreover, while the technology is amenable to various modifications and alternative forms, specific embodiments have been shown by way of example in the drawings and are described in detail below. The intention, however, is not to limit the technology to the particular implementations described. On the contrary, the technology is intended to cover all modifications, equivalents, and alternatives falling within the scope of the technology as defined by the appended claims.

### Detailed Description

Unless the context clearly requires otherwise, throughout the description and the claims, the words "comprise," "comprising," and the like are to be construed in an inclusive sense, as opposed to an exclusive or exhaustive sense; that is to say, in the sense of "including, but not limited to." As used herein, the terms "connected," "coupled," or any variant thereof means any connection or coupling, either direct or indirect, between two or more elements; the coupling or connection between the elements can be physical, logical, electromagnetic, or a combination thereof. Additionally, the words "herein," "above," "below," and words of similar import, when used in this application, refer to this application as a whole and not to any particular portions of this application. Where the context permits, words in the Detailed Description using the singular or plural number may also include the plural or singular number respectively. The word "or," in reference to a list of two or more items, covers all of the following interpretations of the word: any of the items in the list, all of the items in the list, and any combination of the items in the list.

The teachings of the technology provided herein can be applied to other systems, not necessarily the system described below. The elements and acts of the various examples described below can be combined to provide further implementations of the technology. Some alternative implementations of the technology may include not only additional elements to those implementations noted below, but also may include fewer elements.

These and other changes can be made to the technology in light of the following detailed description. While the description describes certain examples of the technology, and describes the best mode contemplated, no matter how detailed the description appears, the technology can be practiced in many ways. Details of the system may vary considerably in its specific implementation, while still being encompassed by the technology disclosed herein. As noted above, particular terminology used when describing certain features or aspects of the technology should not be taken to imply that the terminology is being redefined herein to be restricted to any specific characteristics, features, or aspects of the technology with which that terminology is associated. In general, the terms used in the following claims should not be construed to limit the technology to the specific examples disclosed in the specification, unless the Detailed Description section explicitly defines such terms. Accordingly, the actual scope of the technology encompasses not only the disclosed examples, but also all equivalent ways of practicing or implementing the technology under the claims.

To reduce the number of claims, certain aspects of the technology are presented below in certain claim forms, but the applicant contemplates the various aspects of the technology in any number of claim forms. For example, while some aspect of the technology may be recited as a computer-readable medium claim, other aspects may likewise be embodied as a computer-readable medium claim, or in other forms, such as being embodied in a means-plus-function claim.

In the following description, for the purposes of explanation, numerous specific details are set forth in order to provide a thorough understanding of implementations of the disclosed technology. It will be apparent, however, to one skilled in the art that embodiments of the disclosed technology may be practiced without some of these specific details.

A particle according to some embodiments of the present disclosure comprises an inorganic matrix, a first, polymeric, light-emitting material and a second light-emitting material.

In some embodiments, the second light-emitting material is non-polymeric. Optionally, a non-polymeric light-emitting compound as described herein has a molecular weight of 500 Daltons or less.

In some embodiments, the second light-emitting material is a second polymeric light-emitting material.

In some embodiments, the first, polymeric, light-emitting material has a peak emission wavelength which is shorter that of the second light-emitting material.

The first, polymeric light-emitting material may be configured to transfer energy to the second light-emitting material (e.g. by FRET), allowing for downconversion of light.

Optionally, the first light-emitting material has an absorption spectrum having a peak which at least partially overlaps with an emission spectrum of the second light-emitting material.

In some embodiments the inorganic matrix comprises or consists of a matrix material selected from an oxide, optionally silica, alumina or titanium dioxide.

The first, polymeric, light-emitting material comprises one or more ionic groups, optionally ionic substituents covalently bound to a backbone of the polymer, giving the first light-emitting material one of a net positive and a net negative charge.

The second light-emitting material comprises one or more ionic groups such that the second light-emitting material has the other of a net positive and net negative charge.

By "net charge" of a material as used herein is meant the sum of the charges of anionic or cationic groups covalently bound to or bound into the material, *i.e.* not including the charges of any counterions.

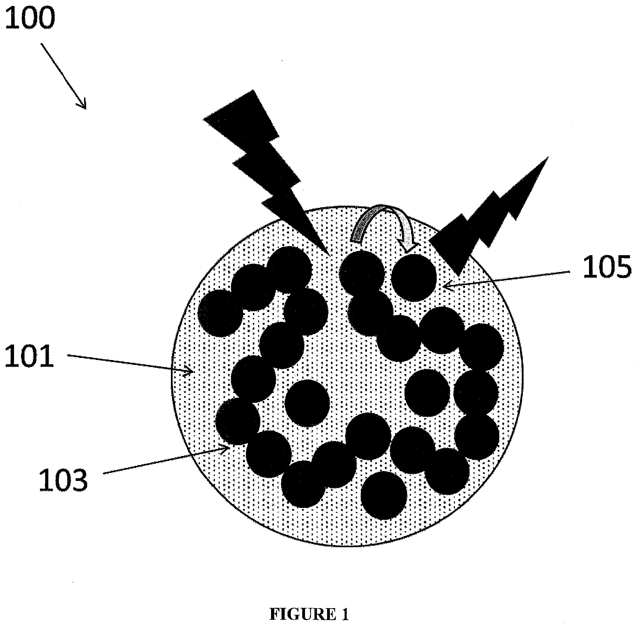

Figure 1 illustrates a particle 100 according to some embodiments. The particle comprises matrix 101, a first, polymeric, light-emitting material 103 and a second, non-polymeric light-emitting compound 105.

Upon excitation of the first light-emitting material 103, for example by electromagnetic radiation at an absorption wavelength of the first light-emitting material, energy may be transferred to the second, non-polymeric light-emitting material 105 by a radiative or non-radiative energy transfer process and light may be emitted from the second light-emitting material. The first and second light-emitting materials, alone or in combination with any further light-emitting materials of the particle, may form a tandem dye.

In some embodiments, the particle may contain three or more light-emitting materials, optionally up to 10 light-emitting materials, each light-emitting material independently being selected from non-polymeric and polymeric light-emitting materials. Optionally, the first light-emitting material is the only polymeric light-emitting material of the particle.

In some embodiments, emission directly from each of the light-emitting materials of the particle may be observed.

In some embodiments, emission may be observed directly from one or more, but not all, light-emitting compounds of the particle as a result of energy transfer between light emitting materials, *e.g.* from the first light-emitting material to the second light-emitting material.

The proportion of total particle emission arising from each light-emitting material may be tuned by selection of the proportion of each light-emitting material of the particle and/or the overlap between emission and absorption spectra of different light-emitting materials.

Optionally, the first light-emitting material is not covalently bound to the matrix material.

Optionally, the second light-emitting material is not covalently bound to the matrix material.

Electrostatic attraction between the opposite charges of the first and second light-emitting materials may prevent or retard washing out of these light-emitting materials, in particular a non-polymeric second light-emitting material from the particle.

The first, polymeric light-emitting material may be less susceptible to washing from the particle due at least in part to its relatively large size. The matrix may comprise a polymer, for example silica, and chains of the polymer may be entangled with, but not covalently bound to, polymer chains of the matrix.

In some embodiments, the particle comprises a biomolecule binding group. The biomolecule binding group may be configured to bind to a target biomolecule. Target biomolecules include without limitation DNA, RNA, peptides, carbohydrates, antibodies, antigens, enzymes, proteins and hormones. It will be understood that the biomolecule binding group may be selected according to the target biomolecule.

Preferably, the particles have a number average diameter of no more than 5000 nm, more preferably no more than 2500nm, 1000nm, 900nm, 800nm, 700nm, 600 nm, 500nm or 400 nm as measured by dynamic light scattering (DLS) using a Malvern Zetasizer Nano ZS. Preferably the particles have a number average diameter of between 5-5000 nm, optionally 10-1000 nm, preferably between 10-500 nm, most preferably between 10-100nm as measured by a Malvern Zetasizer Nano ZS.

Preferably, at least 50 wt% of the total weight of the particle consists of matrix material. Preferably at least 60, 70, 80, 90, 95, 98, 99, 99.5, 99.9 wt% of the total weight of the particle consists of matrix material.

In some embodiments, the particle comprises the first light-emitting material and the second light-emitting material homogeneously distributed through the matrix. In some embodiments the polymeric first light-emitting material, and any other light-emitting polymers mixed with the matrix, are contained within a boundary defined by the matrix material. In some embodiments, one or more light-emitting polymer chains may protrude beyond a boundary defined by the matrix material.

In some embodiments, the particle comprises a core comprising or consisting of the first light-emitting compound and the second light-emitting material and a shell comprising or consisting of the matrix.

### First light-emitting material

The first light-emitting material is a light-emitting polymer. The light-emitting polymer may emit fluorescent light, phosphorescent light or a combination thereof.

The light-emitting polymer may be a homopolymer or may be a copolymer comprising two or more different repeat units.

The light-emitting polymer may comprise light-emitting groups in the polymer backbone, pendant from the polymer backbone or as end groups of the polymer backbone. In the case of a phosphorescent polymer, a phosphorescent metal complex, preferably a phosphorescent iridium complex, may be provided in the polymer backbone, pendant from the polymer backbone or as an end group of the polymer backbone.

The light-emitting polymer may have a non-conjugated backbone or may be a conjugated polymer. By "conjugated polymer" is meant a polymer comprising repeat units in the polymer backbone that are directly conjugated to adjacent repeat units. Conjugated light-emitting polymers include, without limitation, polymers comprising one or more of arylene, heteroarylene and vinylene groups conjugated to one another along the polymer backbone.

The light-emitting polymer may have a linear, branched or crosslinked backbone.

The light-emitting polymer may comprise one or more repeat units in the backbone of the polymer substituted with at least one ionic group. The one or more ionic groups may be the only substituents of said repeat units, or said repeat units may be further substituted with one or more non-ionic substituents, optionally one or more substituents selected from non-ionic polar groups and C₁₋₄₀ hydrocarbyl groups. The repeat unit or repeat units substituted with one or more ionic groups may be the only repeat units of the polymer or the polymer may comprise one or more further co-repeat units wherein the or each co-repeat unit is unsubstituted or is substituted with one or more non-ionic substituents.

The ionic groups of the polymer may enhance solubility of the polymer in polar solvents and may prevent the polymer from assuming a tightly coiled formation as compared to the case where a polymer which is not substituted with any polar groups is placed in a polar solvent.

C₁₋₄₀ hydrocarbyl groups as described herein include, without limitation, C₁₋₂₀ alkyl, unsubstituted phenyl and phenyl substituted with one or more C₁₋₂₀ alkyl groups.

As used herein "non-ionic polar groups" may refer to one more groups which render the light-emitting polymer with a solubility of at least 0.0005 mg/ml in an alcoholic solvent, preferably at least 0.001, more preferably at least 0.01, 0.1, or 1 mg / ml, optionally at least 5 or 10 mg/ml. The solubility is measured at 25°C. Preferably, the alcoholic solvent is a C₁₋₁₀ alcohol, more preferably methanol.

In some embodiments, the polymer backbone may be substituted with non-ionic polar groups of formula -O(R³O)_{q}-R⁴ wherein R³ in each occurrence is a C₁₋₁₀ alkylene group, optionally a C₁₋₅ alkylene group, wherein one or more non-adjacent, non-terminal C atoms of the alkylene group may be replaced with O, R⁴ is H or C₁₋₅ alkyl, and q is at least 1, optionally 1-10. Preferably, q is at least 2. More preferably, q is 2 to 5. The value of q may be the same in all the polar groups of formula -O(R³O)_{q}-R⁴. The value of q may differ between non-ionic polar groups of the same polymer.

By "C₁₋₅ alkylene group" as used herein with respect to R³ is meant a group of formula - (CH₂)_{f}- wherein f is from 1-5.

Preferably, the polymer comprises non-ionic polar groups of formula -O(CH₂CH₂O)_{q}R⁴ wherein q is at least 1, optionally 1-10 and R⁴ is a C₁₋₅ alkyl group, preferably methyl. Preferably, q is at least 2. More preferably, q is 2 to 5, most preferably q is 3.

In some embodiments, the polymer comprises non-ionic polar groups of formula - N(R⁵)₂, wherein R⁵ is H or C₁₋₁₂ hydrocarbyl. Preferably, each R⁵ is a C₁₋₁₂ hydrocarbyl.

Exemplary anionic groups are -COO⁻, a sulfonate group; hydroxide; sulfate; phosphate; phosphinate; or phosphonate.

An exemplary cationic group is -N(R⁵)₃⁺ wherein R⁵ in each occurrence is H or C₁₋₁₂ hydrocarbyl. Preferably, each R⁵ is a C₁₋₁₂ hydrocarbyl.

The charges of at least some ionic groups of the polymer may be balanced by the opposite charge of the first light-emitting compound.

The anionic or cationic group may be monovalent or polyvalent. Preferably, the anionic and cationic groups are monovalent.

The polymer may comprise a plurality of anionic or cationic polar groups wherein the charge of two or more anionic or cationic groups is balanced by a single counterion.

Optionally, the polar groups comprise anionic or cationic groups comprising di- or trivalent counterions.

The counterion is optionally a cation, optionally a metal cation, optionally Li⁺, Na⁺, K⁺, Cs⁺, preferably Cs⁺, or an organic cation, optionally ammonium, such as tetraalkylammonium, ethylmethyl imidazolium or pyridinium.

The counterion is optionally an anion, optionally a halide; a sulfonate group, optionally mesylate or tosylate; hydroxide; carboxylate; sulfate; phosphate; phosphinate; phosphonate; or borate.

In some embodiments, the polymer comprises ionic groups and non-ionic polar groups selected from groups of formula -O(R³O)_{q}-R⁴, groups of formula -N(R⁵)₂ and groups of formula OR⁴. Preferably, the polymer comprises ionic groups of formula -COO⁻and non-ionic polar groups selected from groups of formula -O(CH₂CH₂O)_{q}R⁴ and groups of formula -N(R⁵)₂.

Optionally, the backbone of the light-emitting polymer is a conjugated polymer. Optionally, the backbone of the conjugated light-emitting polymer comprises repeat units of formula (I): 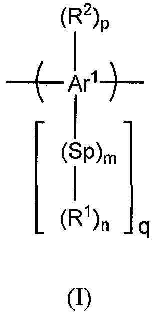 wherein Ar¹ is an arylene group or heteroarylene group; Sp is a spacer group; m is 0 or 1; R¹ independently in each occurrence is a polar group including at least one ionic group; n is 1 if m is 0 and n is at least 1, optionally 1, 2, 3 or 4, if m is 1; R² independently in each occurrence is a non-polar group; p is 0 or a positive integer; q is at least 1, optionally 1, 2, 3 or 4; and wherein Sp, R¹ and R² may independently in each occurrence be the same or different.

Preferably, m is 1 and n is 1-4. Preferably p is 0.

Ar¹ of formula (I) is optionally a C₆₋₂₀ arylene group or a 5-20 membered heteroarylene group. Ar¹ is preferably a C₆₋₂₀ arylene group, optionally phenylene, fluorene, benzofluorene, phenanthrene, naphthalene or anthracene, more preferably fluorene or phenylene, most preferably fluorene.

Sp-(R¹)n may be a branched group, optionally a dendritic group, substituted with polar groups.

Preferably, Sp is selected from:
- C₁₋₂₀ alkylene or phenylene-C₁₋₂₀ alkylene wherein one or more non-adjacent C atoms may be replace with O, S, N or C=O;
- a C₆₋₂₀ arylene or 5-20 membered heteroarylene, more preferably phenylene, which, in addition to the one or more substituents R¹ including one or more ionic groups, may be unsubstituted or substituted with one or more non-polar substituents, optionally one or more C₁₋₂₀ alkyl groups.
"alkylene" as used herein means a branched or linear divalent alkyl chain.
"non-terminal C atom" of an alkyl group as used herein means a C atom other than the methyl group at the end of an n-alkyl group or the methyl groups at the ends of a branched alkyl chain.

More preferably, Sp is selected from:
- C₁₋₂₀ alkylene wherein one or more non-adjacent C atoms may be replaced with O, S or CO; and
- a C₆₋₂₀ arylene or a 5-20 membered heteroarylene, even more preferably phenylene, which may be unsubstituted or substituted with one or more non-polar substituents.

R¹ may be a polar group as described anywhere herein. Preferably, R¹ in each occurrence is independently selected from the group consisting of:
- a polyethylene glycol (PEG) group of formula -O(CH₂CH₂O)_{q}R⁴ wherein q is at least 1, optionally 1-10 and R⁴ is a C₁₋₅ alkyl group, preferably methyl;
- a group of formula -N(R⁵)₂, wherein R⁵ is H or C₁₋₁₂ hydrocarbyl; or
- an anionic group of formula -COO⁻
wherein at least one R¹ is -COO⁻.

In the case where n is at least two, each R¹ may independently in each occurrence be the same or different with the proviso that at least one R¹ is an ionic group. Preferably, each R¹ attached to a given Sp group is different.

In the case where p is a positive integer, optionally 1, 2, 3 or 4, the group R² may be selected from:
- alkyl, optionally C₁₋₂₀ alkyl; and
- aryl and heteroaryl groups that may be unsubstituted or substituted with one or more substituents, preferably phenyl substituted with one or more C₁₋₂₀ alkyl groups;
- a linear or branched chain of aryl or heteroaryl groups, each of which groups may independently be substituted, for example a group of formula -(Ar³)ₛ wherein each Ar³ is independently an aryl or heteroaryl group and s is at least 2, preferably a branched or linear chain of phenyl groups each of which may be unsubstituted or substituted with one or more C₁₋₂₀ alkyl groups; and
- a crosslinkable-group, for example a group comprising a double bond such and a vinyl or acrylate group, or a benzocyclobutane group.

Preferably, each R², where present, is independently selected from C₁₋₄₀ hydrocarbyl, and is more preferably selected from C₁₋₂₀ alkyl; unsubstituted phenyl; phenyl substituted with one or more C₁₋₂₀ alkyl groups; and a linear or branched chain of phenyl groups, wherein each phenyl may be unsubstituted or substituted with one or more substituents.

A polymer as described herein may comprise or consist of only one form of the repeating unit of formula (I) or may comprise or consist of two or more different repeat units of formula (I).

Optionally, the polymer comprising one or more repeat units of formula (I) is a copolymer comprising one or more co-repeat units.

If co-repeat units are present then the repeat units of formula (I) may form between 0.1-99 mol % of the repeat units of the polymer, optionally 50-99 mol % or 80-99 mol %. Preferably, the repeat units of formula (I) form at least 50 mol% of the repeat units of the polymer, more preferably at least 60, 70, 80, 90, 95, 98 or 99 mol%. Most preferably the repeat units of the polymer consist of one or more repeat units of formula (I).

The repeat unit of the light-emitting polymer, in the case where the polymer is a homopolymer, or one or more of the different repeat units of the polymer, in the case wherein the polymer is a copolymer, may be selected to produce a desired colour of emission of the polymer.

The light-emitting polymer may emit light having a peak wavelength in the range of 350-1000 nm.

A blue light-emitting polymer of a particle as described herein may have a photoluminescence spectrum with a peak of no more than 500 nm, preferably in the range of 400-500 nm, optionally 400-490 nm.

A green light-emitting polymer of a particle as described herein may have a photoluminescence spectrum with a peak of more than 500 nm up to 580 nm, optionally more than 500 nm up to 540 nm.

A red light-emitting polymer of a particle as described herein may have a photoluminescence spectrum with a peak of no more than more than 580 nm up to 630 nm, optionally 585 nm up to 625 nm.

The photoluminescence spectrum of light-emitting materials as described herein may be as measured using an Ocean Optics 2000+ spectrometer.

The light-emitting polymer may have an absorption spectrum with a peak in the range of 300-900 nm.

The light-emitting polymer may have a Stokes shift in the range of 10-850 nm, optionally 350-850 nm.

UV/vis absorption spectra of light-emitting polymers as described herein may be as measured in methanol using a Cary 5000 UV-vis-IR spectrometer.

The polymer may be non-conjugated or conjugated.

The polymer is preferably a conjugated polymer comprising repeat units of formula (I) conjugated to one another and / or conjugated to aromatic or heteroaromatic groups of co-repeat units adjacent to the repeat units of formula (I). Exemplary conjugated polymers include polymers comprising arylenevinylene repeat units; arylene repeat units; heteroarylene repeat units; amine repeat units; and combinations thereof.

If present, the or each co-repeat unit may be unsubstituted or substituted with one or more non-ionic substituents, optionally one or more repeat units comprising or consisting of one or more groups selected from C₆₋₂₀ arylene groups and 5-20 membered heteroarylene groups, wherein each of said arylene or heteroarylene groups independently in each occurrence may be unsubstituted or substituted with one or more non-ionic substituents.

Arylene repeat units of the polymer include, without limitation, fluorene, preferably a 2,7-linked fluorene; phenylene, preferably a 1,4-linked phenylene; naphthalene, anthracene, indenofluorene, phenanthrene and dihydrophenanthrene repeat units. Arylene co-repeat units may be selected from repeat units of formulae (III)-(VI): 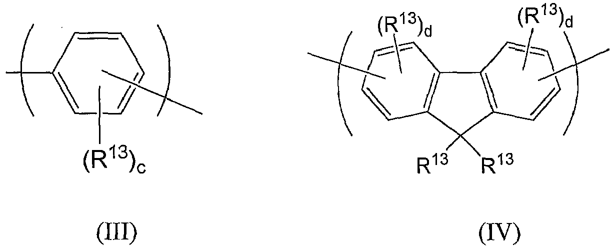 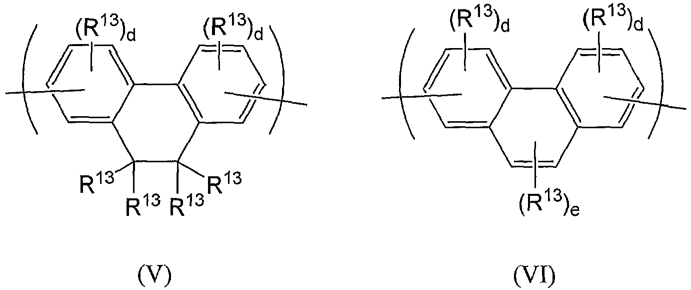 wherein R¹³ in each occurrence is independently a substituent; c is 0, 1, 2, 3 or 4, preferably 1 or 2; each d is independently 0, 1,2 or 3, preferably 0 or 1; and e is 0, 1 or 2, preferably 2.

Repeat units comprising or consisting of one or more unsubstituted or substituted 5-20 membered heteroarylene groups in the polymer backbone include, without limitation, thiophene repeat units, bithiophene repeat units, benzothiadiazole repeat units, and combinations thereof. Exemplary heteroarylene co-repeat units include repeat units of formulae (VII), (VIII) and (IX): 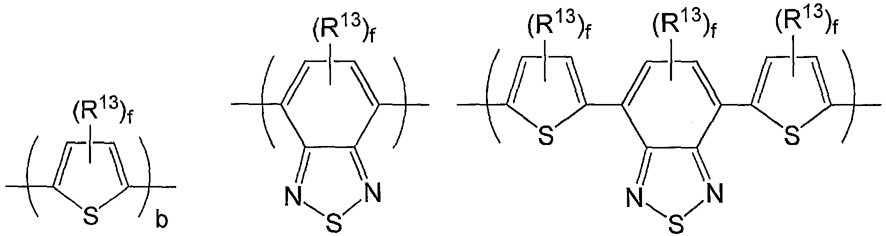 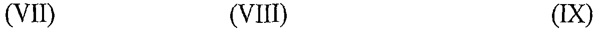 wherein R¹³ in each occurrence is independently a substituent and f is 0, 1 or 2.

R¹³ in each occurrence may independently be a group comprising or consisting of a ionic group, optionally an ionic substituent -(Sp)ₘ-(R¹)ₙ, or a non-ionic substituent R² wherein Sp, m and R¹ are as described with reference to Formula (I).

Arylene repeat units or heteroarylene repeat units substituted with one or more polar groups, optionally repeat units of formulae (III)-(IX) substituted with one groups of formula -(Sp)ₘ-(R¹)ₙ, are repeat units of formula (I).

Arylene repeat units or heteroarylene repeat units, optionally repeat units of formulae (III)-(IX), which are unsubstituted or substituted only with one or more non-polar groups, are co-repeat units of the polymer.

Amine repeat units of the polymer may have formula (XII): 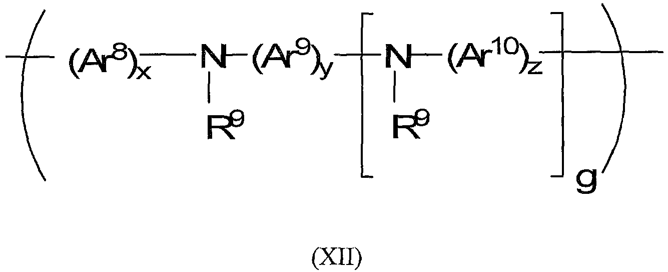 wherein Ar⁸, Ar⁹ and Ar¹⁰ in each occurrence are independently selected from substituted or unsubstituted aryl or heteroaryl, g is 0, 1 or 2, preferably 0 or 1, R¹³ independently in each occurrence is a substituent, and x, y and z are each independently 1, 2 or 3.

R⁹, which may be the same or different in each occurrence when g is 1 or 2, is preferably selected from the group consisting of alkyl, optionally C₁₋₂₀ alkyl, Ar¹¹ and a branched or linear chain of Ar¹¹ groups wherein Ar¹¹ in each occurrence is independently substituted or unsubstituted aryl or heteroaryl.

Any two aromatic or heteroaromatic groups selected from Ar⁸, Ar⁹, and, if present, Ar¹⁰ and Ar¹¹ that are directly bound to the same N atom may be linked by a direct bond or a divalent linking atom or group. Preferred divalent linking atoms and groups include O, S; substituted N; and substituted C.

Ar⁸ and Ar¹⁰ are preferably C₆₋₂₀ aryl, more preferably phenyl that may be unsubstituted or substituted with one or more substituents.

In the case where g = 0, Ar⁹ is preferably C₆₋₂₀ aryl, more preferably phenyl, that may be unsubstituted or substituted with one or more substituents.

In the case where g = 1, Ar⁹ is preferably C₆₋₂₀ aryl, more preferably phenyl or a polycyclic aromatic group, for example naphthalene, perylene, anthracene or fluorene, that may be unsubstituted or substituted with one or more substituents.

R⁹ is preferably Ar¹¹ or a branched or linear chain of Ar¹¹ groups. Ar¹¹ in each occurrence is preferably phenyl that may be unsubstituted or substituted with one or more substituents.

Exemplary groups R⁹ include the following, each of which may be unsubstituted or substituted with one or more substituents, and wherein * represents a point of attachment to N: 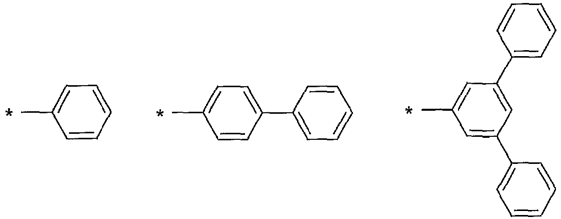 x, y and z are preferably each 1.

Ar⁸, Ar⁹, and, if present, Ar¹⁰ and Ar¹¹ are each independently unsubstituted or substituted with one or more, optionally 1, 2, 3 or 4, substituents.

Substituents may independently be a group comprising or consisting of a polar group, optionally a polar substituent -(Sp)ₘ-(R¹)ₙ, or a non-polar substituent R² wherein Sp, m, R¹ and R² are as described with reference to Formula (I).

Preferred substituents of Ar⁸, Ar⁹, and, if present, Ar¹⁰ and Ar¹¹ are C₁₋₄₀ hydrocarbyl, preferably C₁₋₂₀ alkyl.

Preferred repeat units of formula (XII) include unsubstituted or substituted units of formulae (XII-1), (XII-2) and (XII-3):

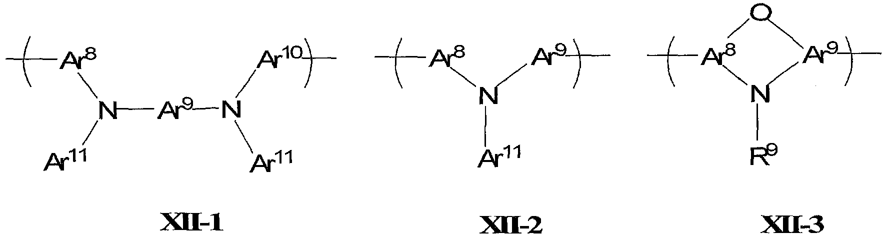

Preferably, a polymer comprising a repeat unit of formula (XII) further comprises one or more arylene repeat units, optionally one or more arylene repeat units selected from formulae (III)-(IX). Optionally, 0.1 - 50 mol % of a light-emitting polymer are one or more repeat units of formula (XII). Optionally, the repeat units of a light-emitting polymer comprise or consist of one or more repeat units of formula (XII) and one or more arylene repeat units, optionally one or more repeat units of formulae (III)-(IX).

The polymer may contain one or more repeat units selected from formulae (XIII-XVII): 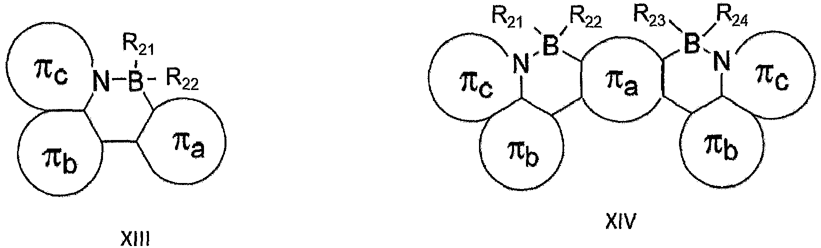 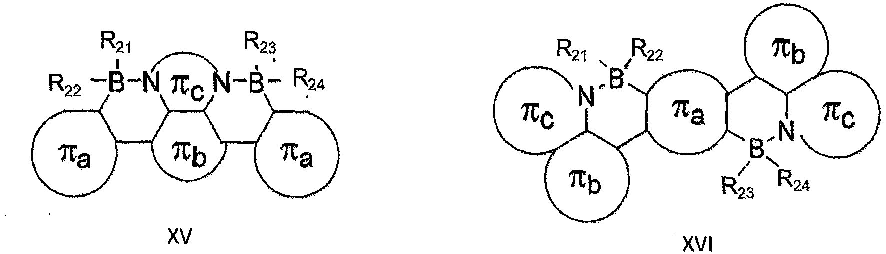 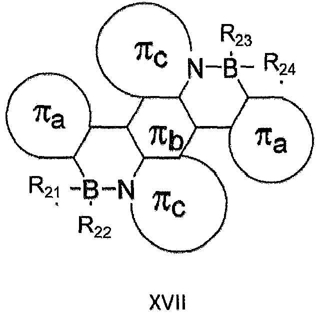
R²¹, R²², R²³ and R²⁴ are each independently selected from hydrogen, fluoro, chloro, bromo, substituted or unsubstituted (1-10C)alkyl, substituted or unsubstituted (2-10C)alkenyl, substituted or unsubstituted (2-10C)alkynyl, substituted or unsubstituted aryl, substituted or unsubstituted heteroaryl, substituted or unsubstituted amino, substituted or unsubstituted (1-10C)alkoxy, substituted or unsubstituted arylalkoxy, and hydroxyl;
each πa independently represents a π-conjugated π-donor ring system formed from one, two, three or four 6-membered aryl or 5 to 6-membered heteroaryl rings; each πb independently represents a π-conjugated 6-membered aryl or 5-6 membered heteroaryl ring;
each πc independently represents a π-conjugated 5-6 membered heteroaryl ring, which when taken in combination with πb, forms a π-acceptor ring system; wherein:
   any or all of the rings forming πa, πb and πc may be independently optionally substituted with one more ring substituents selected from halo, (1-20C)alkyl, (2-20C)alkenyl, (2-20C)alkynyl, aryl, heteroaryl, cycloalkyl, heterocyclyl, carboxyl, phosphoryl, sulfonyl, hydroxyl, (1-20C)alkoxy, nitro, amino, mercapto, silyl, siloxy, azido, boronic acid group, sulfonic acid group, hydroxamic acid group, cyanoacrylate group, and dioxocyclobutenyl group having at least one functional group selected from the group consisting of a carboxyl, phosphoryl, sulfonyl, hydroxyl, alkoxy, nitro, amino, mercapto, silyl, siloxy, azido, boronic acid group, sulfonic acid group, hydroxamic acid group and cyanoacrylate group.

The repeat unit of formula (XIII) may be bound into the polymer backbone by a bond to πa and one of πb and πc.

The repeat units of formula (XIV) and (XVI) may be bound into the polymer backbone by a bond to πb or πc on one side of πa and by a bond to πb or πc on the other side of πa.

The repeat unit of formula (XV) may be bound into the polymer backbone by a bond to each one of πa.

The repeat unit of formula (XVII) may be bound into the polymer backbone by a bond to each one of πa or by a bond to each one of πc.

Exemplary repeat units of formulae (XIII)-(XVII) include:

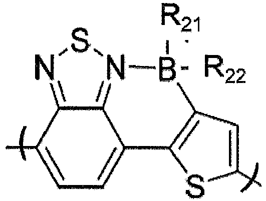

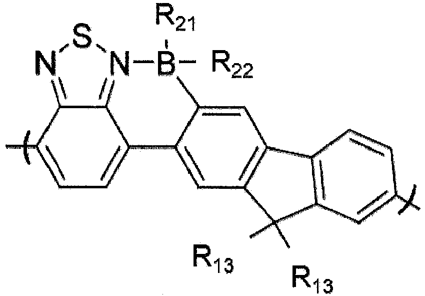

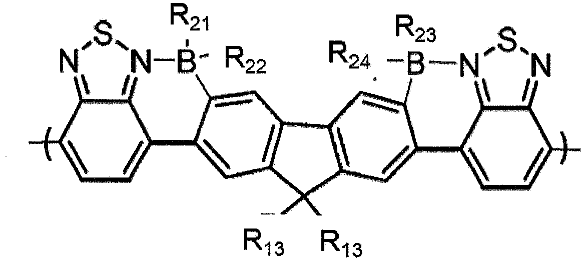

In the case of a phosphorescent conjugated polymer a phosphorescent group, preferably a metal complex, more preferably an iridium complex, may be provided in the main chain, in a side group and / or as an end group of the polymer. An exemplary conjugating repeat unit comprising an iridium complex has formula:

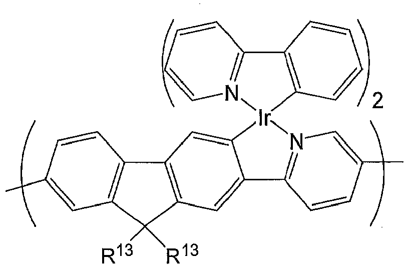

Preferably, the repeat unit of formula (I) is a repeat unit of formula (Ia): 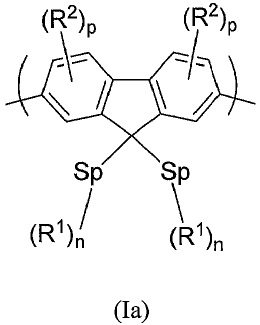 wherein R², p, Sp, R¹ and n are independently in each occurrence as described in relation to the repeat unit of formula (I). Preferably, n in each occurrence is 2. Preferably p in each occurrence is 0.

An exemplary repeat unit of formula (Ia) is:

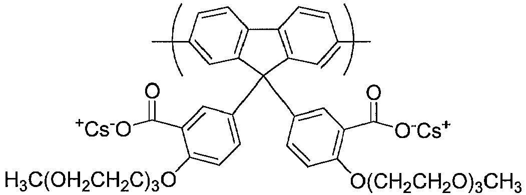

The non-polymeric emitter: polymer ratio may be in the range of about 1 : 0.5 - 1 : 200, optionally about 1 : 1 - 1 : 100.

The polystyrene-equivalent number-average molecular weight (Mn) measured by gel permeation chromatography of the polymers described herein, preferably the light-emitting polymers described herein may be in the range of about 1×10³ to 1×10⁸, and preferably 1×10⁴ to 5×10⁶. The polystyrene-equivalent weight-average molecular weight (Mw) of the polymers described herein may be 1×10³ to 1×10⁸, and preferably 1×10⁴ to 1×10⁷.

### Second light-emitting material

The second light-emitting material may be a polymeric material, optionally a polymer selected from light-emitting polymers described above.

Preferably, the second light-emitting material is a non-polymeric light-emitting material. A non-polymeric light-emitting material as described herein optionally has a molecular weight of 500 Daltons or less.

Preferably, the non-polymeric light-emitting material is monodisperse.

The second light-emitting material may have a net ionic charge opposite to a net ionic charge of the first light-emitting material.

Exemplary non-polymeric light-emitting materials include, without limitation, fluoresceins and salts thereof; rhodamines, for example Rhodamine 6G and Rhodamine 110 chloride; coumarins; boron-dipyrromethenes (BODIPYs); naphthalimides; perylenes; benzanthrones; benzoxanthrones; and benzothiooxanthrones, each of which may be unsubstituted or substituted with one or more substituents. Exemplary substituents are chlorine, alkyl amino; phenylamino; and hydroxyphenyl.

### Matrix

The inorganic matrix may be an oxide, optionally silica, alumina or titanium dioxide.

Preferably, the inorganic matrix is not covalently bound to the first or second light-emitting material. Accordingly, there is no need for the matrix material, the polymer and / or the non-polymeric light-emitting material to be substituted with reactive groups for forming such covalent bonds, e.g. during formation of the particles.

In some embodiments, a silica matrix as described herein may be formed by polymerisation of a silica monomer in the presence of the non-polymeric light-emitting material and the polymer.

In some embodiments, the polymerisation comprises treating a solution comprising silica monomer, the first light-emitting material and the second light-emitting material with a base, or by adding a solution of silica monomer to a solution of the first and second light-emitting materials and a base. Optionally, the solvent of the solutions are water, one or more C₁₋₁₀ alcohols or a combination thereof.

In some embodiments, the process comprises polymerising silica monomer in a solution of the matrix monomer and the first and second light-emitting materials under acidic conditions.

Polymerising a matrix monomer in the presence of a light-emitting polymer may result in one or more chains of the polymer encapsulated within the particle and / or one or more chains of the polymer extending through a particle.

The particles may be formed in a one-step polymerisation process.

Optionally, the silica monomer is an alkoxysilane, preferably a trialkoxy or tetra-alkoxysilane, optionally a C₁₋₁₂ trialkoxy or tetra-alkoxysilane, for example tetraethyl orthosilicate. The silica monomer may be substituted only with alkoxy groups or may be substituted with one or more groups.

In some embodiments, a biomolecule binding group is bound to a surface of the particle. The biomolecule binding group may be bound directly to the surface of the particle group or bound through a surface binding group. The surface binding group may comprise polar groups. Optionally, the surface binding group comprises a polyether chain. By "polyether chain" as used herein is meant a chain having two or more ether oxygen atoms.

Silica at the surface of the particles may be reacted to form a group at the surface capable of binding to a biomolecule binding group. Optionally, silica at the surface is reacted with a siloxane.

The biomolecule binding group may be selected from the group consisting of: DNA, RNA, peptides, carbohydrates, antibodies, antigens, enzymes, proteins and hormones. The biomolecule binding group may be selected according to a target biomolecule to be detected. The target biomolecule may or may not be a biomolecule, e.g. a protein, at a surface of a cell.

A preferred biomolecule binding group is biotin. In some embodiments, the biotin biomolecule binding group binds directly to a target analyte.

In some embodiments, the biotin biomolecule binding group is bound to a protein having a plurality of biotin binding sites, preferably streptavidin, neutravidin, avidin or a recombinant variant or derivative thereof and biotinylated biomolecule having a second biotin group is bound to the same protein. The biotinylated biomolecule may be selected according to the target analyte. The biotinylated biomolecule may comprise an antigen binding fragment, e.g. an antibody, which may be selected according to a target antigen.

### Colloids

The particles may be provided as a colloidal suspension comprising the particles suspended in a liquid. Preferably, the liquid is selected from water, C₁₋₁₀ alcohols and mixtures thereof. Preferably, the particles form a uniform (non-aggregated) colloid in the liquid.

The liquid may be a solution comprising salts dissolved therein, optionally a buffer solution.

The concentration of the particles in the colloidal suspension is preferably in the range of 0.1-20 mg / mL, optionally 5-20 mg / mL.

### Applications

The particles of the present disclosure may be fluorescent or phosphorescent. Preferably the particles are fluorescent. Preferably the particles are for use as a fluorescent probe for detecting a biomolecule or for labelling a biomolecule. In some embodiments, the particles may be used as a fluorescent probe in an immunoassay such as a lateral flow or solid state immunoassay. Optionally the particles are for use in fluorescence microscopy, flow cytometry, next generation sequencing, in-vivo imaging, or any other application where a light-emitting marker configured to bind to a target analyte is brought into contact with a sample to be analysed. The applications can be medical, veterinary, agricultural or environmental applications whether involving patients (where applicable) or for research purposes.

In use the biomolecule binding group of the particles may bind to target biomolecules which include without limitation DNA, RNA, peptides, carbohydrates, antibodies, antigens, enzymes, proteins and hormones. It will be understood that the biomolecule binding group may be selected according to the target biomolecule.

A sample to be analysed may brought into contact with the particles, for example the particles in a colloidal suspension.

In some embodiments, the sample following contact with the particles is analysed by flow cytometry. In flow cytometry, the particles are irradiated by at least one wavelength of light, optionally two or more different wavelengths, e.g. one or more wavelengths including at least one of 355, 405, 488, 562 and 640 nm. Light emitted by the particles may be collected by one or more detectors. Detectors may be selected from, without limitation, photomultiplier tubes and photodiodes. To provide a background signal for calculation of a staining index, measurement may be made of particles mixed with cells which do not bind to the particles

In some embodiments, the target biomolecule may be immobilised on a surface which is brought into contact with the particles.

In some embodiments, the particles may be stored in a dry, optionally lyophilised, form. In some embodiments, the particles may be stored in a frozen form.

### Example 1

### Nanoparticle formation

A nanoparticle of a silica matrix having blue light-emitting polymer LEP1 and green-emitting small molecule Rhodamine G6 dispersed therein was formed.

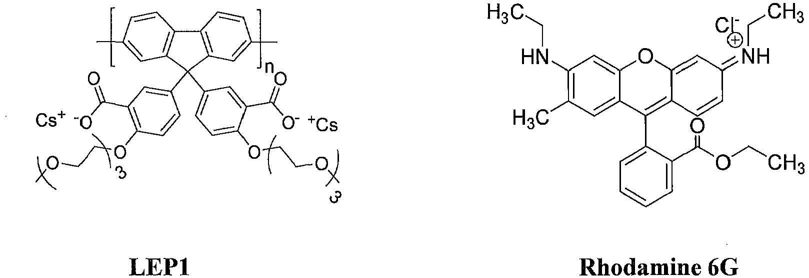

To a solution of **LEP1** in anhydrous methanol (2 mg mL⁻¹, 1 mL) was added a solution of Rhodamine 6G in the same solvent (2 mg mL⁻¹, 1 mL), giving an LEP1: Rhodamine 6G weight ratio of 1:1.

To this mixture was added ammonium hydroxide solution (28-30%, 150 uL), followed by rapid addition of a solution of tetraethylorthosilicate (200 uL) in anhydrous methanol (500 uL) with stirring at room temperature. The reaction mixture was stirred at room temperature for 1 hour, after which time the resultant dispersion was centrifuged for 3 min at 14000 rpm and the supernatant was removed by decantation. The solid nanoparticle pellet thus isolated was redispersed by adding 2 mL of anhydrous methanol and sonicating in a bath sonicator for 5 minutes. Once fully redispersed, the solids were again isolated by centrifugation and decantation of the supernatant and this methanol wash step was repeated twice more to give Nanoparticle Example 1 (NP1).

After the final wash step the pellet was resuspended in 2 mL of methanol for analysis.

### Example 2

Nanoparticle Example 2 (NP2) was formed as described for Example 1 except that 0.2 mg mL⁻¹ of the rhodamine 6G was used, giving an LEP1: rhodamine 6G mass ratio in the starting reaction mixture of 10:1.

### Example 3

Nanoparticle Example 3 (NP3) was formed as described for Example 1 except that 0.02 mg mL⁻¹ of the rhodamine 6G was used, giving an LEP1: rhodamine 6G mass ratio in the starting reaction mixture of 100:1.

UV/vis spectra in methanol were recorded using a Cary 5000 UV-vis-IR spectrometer. Photoluminescence spectra in the same dispersant were recorded using an Olympus BX62 microscope, with a mercury short arc lamp (λₑₓ = 365 nm) for excitation and an Ocean Optics 2000+ spectrometer as the detector.

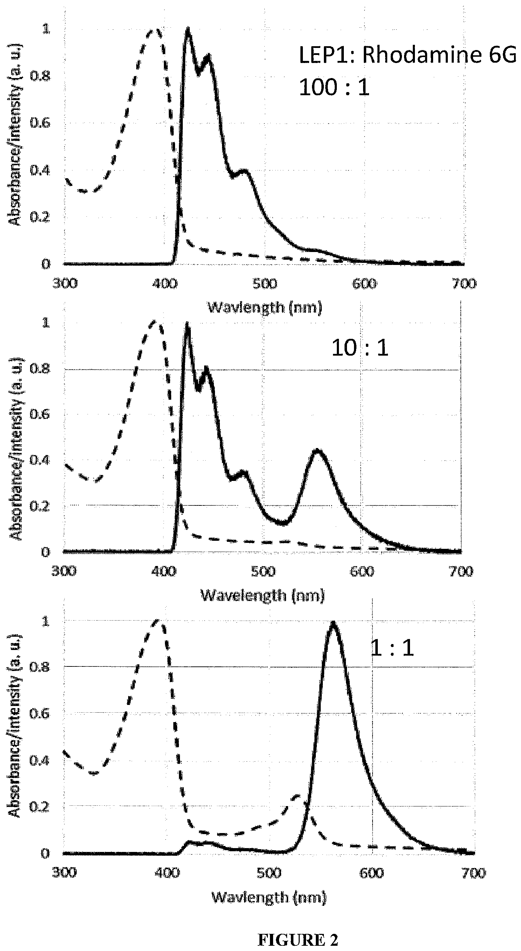

With reference to Figure 2, at higher Rhodamine 6G mass ratios the proportion of emission from blue-emitting LEP1 (peak at ~430 nm) decreases, with a corresponding increase in emission from Rhodamine 6G (peak at ~570 nm) which indicates that energy is being transferred from LEP1 and downconverted by Rhodamine 6G.

A Malvern Zetasizer Nano S was used to determine the size and polydispersity of the nanoparticles as a dispersion in methanol at the as-purified concentration.

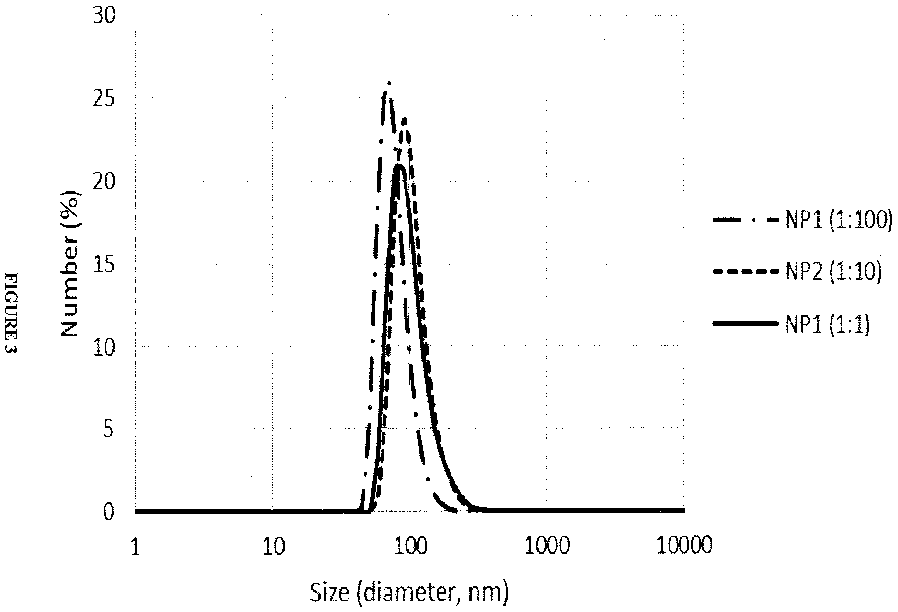

With reference to Figure 3, the LEP1: rhodamine 6G weight ratio had little or no effect on the size of the nanoparticles.

### Examples 4-5

Nanoparticles were prepared as described in Example 1 except that Rhodamine 6G was replaced in Examples 4 and 5 with, respectively, fluorescein sodium salt and rhodamine 110 chloride.

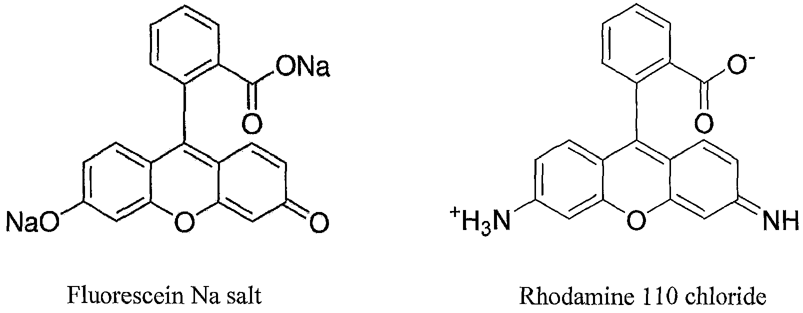

Nanoparticles NP1 of Example 1 and nanoparticles of Examples 4 and 5 were washed three times with methanol.

The LEP1 : Rhodamine 6G mass ratio of NP1 was found to be comparable to the 1 : 1 ratio in the starting mixture whereas some of the small molecule emitters of Examples 4 and 5 were found to have been washed out of the nanoparticles. Upon successive washing of nanoparticles of Examples 4 and 5, the light emitted by the nanoparticles moved closer to that of the light-emitting polymer alone.

Without wishing to be bound by any theory, it is believed that electrostatic attraction between the anionic substituent of LEP1 and the cationic group of Rhodamine 6G may prevent or retard washing out of the non-polymeric light-emitting material.

## Claims

1. A particle comprising an inorganic matrix material; a first light-emitting material; and a second light-emitting material, wherein the first light-emitting material is a light-emitting polymer and wherein the first light-emitting material has one of a net positive and net negative ionic charge, and the second light-emitting material has the other of net positive or net negative ionic charge.

2. The particle according to claim 1 wherein the second light-emitting material is a non-polymeric material.

3. The particle according to claim 1 or 2 wherein the light-emitting polymer is a conjugated polymer.

4. The particle according to claim 3 wherein the conjugated polymer comprises a repeat unit of formula (I): 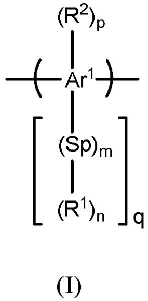 wherein Ar¹ is an arylene group or heteroarylene group; Sp is a spacer group; m is 0 or 1; R¹ independently in each occurrence is a polar group wherein at least one R¹ is an ionic group; n is 1 if m is 0 and n is at least 1 if m is 1; R² independently in each occurrence is a non-polar group; p is 0 or a positive integer; q is at least 1; and wherein Sp, R¹ and R² independently in each occurrence are the same or different.

5. The particle according to claim 4 wherein the repeat unit of formula (I) is a repeat unit of formula (Ia): 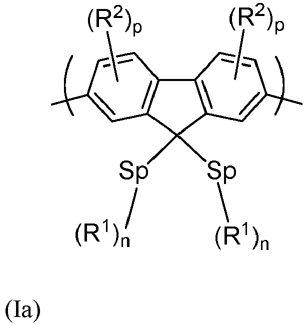 wherein R², p, Sp, R¹ and n are independently in each occurrence as defined in claim 6.

6. The particle according to any one of the preceding claims wherein the first light-emitting material is configured to transfer energy to the second light-emitting material.

7. The particle according to any one of the preceding claims wherein the particle comprises a biomolecule binding group configured to bind to a target biomolecule.

8. The particle according to any one of the preceding claims wherein the inorganic matrix comprises silica.

9. A colloidal suspension comprising particles according to any one of the preceding claims suspended in a liquid.

10. A colloidal suspension according to claim 9 wherein the liquid is a protic liquid.

11. A process for preparing a particle according to claim 8, comprising formation of the silica by polymerisation of a silica monomer in the presence of the first light-emitting material and the second light-emitting material.

12. A method of marking a biomolecule, the method comprising the step of binding the biomolecule to a particle according to any one of claims 1-8.

13. An assay method for a target analyte comprising contacting a sample with light-emitting marker particles according to any one of claim 1-8 and determining any binding of the target analyte to the light-emitting marker.

14. An assay method according to claim 13 wherein the sample contacted with the light-emitting marker particles is analysed by flow cytometry.

15. An assay method according to claim 14 wherein an amount of target analyte bound to the light-emitting marker particles is determined, optionally wherein the sample comprises a mixture of cells and one or more different types of target cells bound to the light-emitting marker are identified and / or quantified.

## Patentansprüche

1. Teilchen, umfassend ein anorganisches Matrixmaterial; ein erstes lichtemittierendes Material; und ein zweites lichtemittierendes Material, wobei das erste lichtemittierende Material ein lichtemittierendes Polymer ist und wobei das erste lichtemittierende Material eines von einer Netto-Positiv- und einer Netto-Negativ-Ionenladung aufweist und das zweite lichtemittierende Material das andere von Netto-Positiv- oder Netto-Negativ-Ionenladung aufweist.

2. Teilchen nach Anspruch 1, wobei das zweite lichtemittierende Material ein nichtpolymeres Material ist.

3. Teilchen nach Anspruch 1 oder 2, wobei das lichtemittierende Polymer ein konjugiertes Polymer ist.

4. Teilchen nach Anspruch 3, wobei das konjugierte Polymer eine Wiederholungseinheit der Formel (I) umfasst: 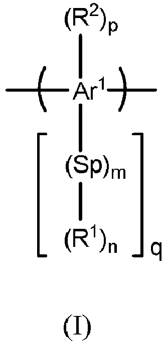 wobei Ar¹ eine Arylengruppe oder Heteroarylengruppe ist; Sp eine Spacer-Gruppe ist; m 0 oder 1 ist; R¹ unabhängig bei jedem Vorkommen eine polare Gruppe ist, wobei zumindest ein R¹ eine Ionengruppe ist; n 1 ist, wenn m 0 ist und n zumindest 1 ist, wenn m 1 ist; R² unabhängig bei jedem Vorkommen eine nicht-polare Gruppe ist; p 0 oder eine positive ganze Zahl ist; q zumindest 1 ist; und wobei Sp, R¹ und R² unabhängig bei jedem Vorkommen gleich oder verschieden sind.

5. Teilchen nach Anspruch 4, wobei die Wiederholungseinheit der Formel (I) eine Wiederholungseinheit der Formel (Ia) ist: 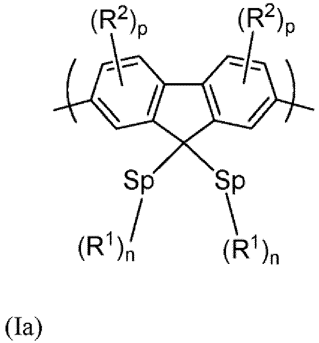 wobei R², p, Sp, R¹ und n unabhängig bei jedem Vorkommen wie in Anspruch 6 definiert sind.

6. Teilchen nach einem der vorhergehenden Ansprüche, wobei das erste lichtemittierende Material konfiguriert ist, um Energie auf das zweite lichtemittierende Material zu übertragen.

7. Teilchen nach einem der vorhergehenden Ansprüche, wobei das Teilchen eine Biomolekülbindungsgruppe umfasst, die konfiguriert ist, um an ein Zielbiomolekül zu binden.

8. Teilchen nach einem der vorhergehenden Ansprüche, wobei die anorganische Matrix Siliziumdioxid umfasst.

9. Kolloidale Suspension, umfassend Teilchen nach einem der vorhergehenden Ansprüche suspendiert in einer Flüssigkeit.

10. Kolloidale Suspension nach Anspruch 9, wobei die Flüssigkeit eine protische Flüssigkeit ist.

11. Prozess zur Herstellung eines Teilchens nach Anspruch 8, umfassend Bildung des Siliziumdioxids durch Polymerisation eines Siliziumdioxidmonomers in der Gegenwart des ersten lichtemittierenden Materials und des zweiten lichtemittierenden Materials.

12. Verfahren zum Markieren eines Biomoleküls, wobei das Verfahren den Schritt des Bindens des Biomoleküls an ein Teilchen nach einem der Ansprüche 1-8 umfasst.

13. Assay-Verfahren für einen Zielanalyten, umfassend Kontaktieren einer Probe mit lichtemittierenden Markerteilchen nach einem von Anspruch 1-8 und Bestimmen von beliebiger Bindung des Zielanalyten an den lichtemittierenden Marker.

14. Assay-Verfahren nach Anspruch 13, wobei die Probe, die mit den lichtemittierenden Markerteilchen kontaktiert wird, durch Durchflusszytometrie analysiert wird.

15. Assay-Verfahren nach Anspruch 14, wobei eine Menge an Zielanalyt, die an die lichtemittierenden Markerteilchen gebunden ist, bestimmt wird, wobei optional die Probe eine Mischung aus Zellen umfasst und eine oder mehrere verschiedene Arten von Zielzellen, die an den lichtemittierenden Marker gebunden sind, identifiziert und / oder quantifiziert werden.

## Revendications

1. Particule comprenant un matériau de matrice inorganique ; un premier matériau électroluminescent ; et un second matériau électroluminescent, ledit premier matériau électroluminescent étant un polymère électroluminescent et ledit premier matériau électroluminescent comprenant l'une d'une charge ionique positive nette et d'une charge ionique négative nette, et ledit second matériau électroluminescent possédant l'autre d'une charge ionique positive nette ou d'une charge ionique négative nette.

2. Particule selon la revendication 1, ledit second matériau électroluminescent étant un matériau non polymère.

3. Particule selon la revendication 1 ou 2, ledit polymère électroluminescent étant un polymère conjugué.

4. Particule selon la revendication 3, ledit polymère conjugué comprenant un motif de répétition de formule (I) : 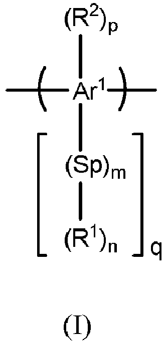 Ar¹ étant un groupe arylène ou un groupe hétéroarylène ; Sp étant un groupe espaceur ; m étant 0 ou 1 ; R¹ indépendamment dans chaque occurrence étant un groupe polaire au moins un R¹ étant un groupe ionique ; n valant 1 si m vaut 0 et n valant au moins 1 si m vaut 1 ; R² indépendamment dans chaque occurrence étant un groupe non polaire ; p étant 0 ou un entier positif ; q valant au moins 1 ; et Sp, R¹ et R² indépendamment dans chaque occurrence étant identiques ou différents.

5. Particule selon la revendication 4, ledit motif de répétition de formule (I) étant un motif de répétition de formule (Ia) : 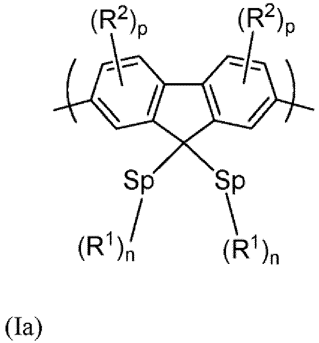 R², p, Sp, R¹ et n étant indépendamment dans chaque occurrence tels que définis dans la revendication 6.

6. Particule selon l'une quelconque des revendications précédentes, ledit premier matériau électroluminescent étant configuré pour transférer de l'énergie au second matériau électroluminescent.

7. Particule selon l'une quelconque des revendications précédentes, ladite particule comprenant un groupe de liaison à une biomolécule conçu pour se lier à une biomolécule cible.

8. Particule selon l'une quelconque des revendications précédentes, ladite matrice inorganique comprenant de la silice.

9. Suspension colloïdale comprenant des particules selon l'une quelconque des revendications précédentes en suspension dans un liquide.

10. Suspension colloïdale selon la revendication 9, ledit liquide étant un liquide protique.

11. Processus permettant la préparation d'une particule selon la revendication 8, comprenant la formation de la silice par polymérisation d'un monomère de silice en présence du premier matériau électroluminescent et du second matériau électroluminescent.

12. Procédé de marquage d'une biomolécule, le procédé comprenant l'étape de liaison de la biomolécule à une particule selon l'une quelconque des revendications 1 à 8.

13. Procédé de dosage d'une substance à analyser cible comprenant la mise en contact d'un échantillon avec des particules marqueurs électroluminescentes selon l'une quelconque des revendications 1 à 8 et la détermination de toute liaison de la substance à analyser cible au marqueur électroluminescent.

14. Procédé d'analyse selon la revendication 13, ledit échantillon mis en contact avec les particules marqueurs électroluminescentes étant analysé par cytométrie en flux.

15. Procédé d'analyse selon la revendication 14, une quantité de substance à analyser cible lié aux particules de marqueur électroluminescent étant déterminée, éventuellement ledit échantillon comprenant un mélange de cellules et un ou plusieurs types différents de cellules cibles liées au marqueur électroluminescent étant identifiés et/ou quantifiés.
